# EUROPÄISCHE PATENTSCHRIFT

(11) **EP 1 958 564 B1**
(45) Veröffentlichungstag und Bekanntmachung des Hinweises auf die Patenterteilung: **08.06.2011**
(21) Anmeldenummer: 08001973.0
(22) Anmeldetag: 02.02.2008
(51) Int. Cl.: A61B 1/05

(54) **Videoendoskop**
Video endoscope
Endoscope vidéo

(30) Priorität: 16.02.2007 DE 102007009292
(43) Veröffentlichungstag der Anmeldung: 20.08.2008
(73) Patentinhaber: Karl Storz GmbH & Co. KG, 78532 Tuttlingen (DE)
(72) Erfinder: Irion, Klaus M.; Dr.-Ing., 78576 Emmingen-Liptingen (DE); Schwarz, Peter, 78532 Tuttlingen (DE); Graf, Christian, 78576 Emmingen-Liptingen (DE); Rockenstiehl, Frank, 72336 Balingen (DE)
(74) Vertreter: Heuckeroth, Volker

(56) Entgegenhaltungen:
- EP-A- 0 904 725
- WO-A-02/102224
- DE-B3-102004 023 866
- DE-U1- 20 113 031
- FR-A- 2 800 983
- US-A- 4 858 001
- US-A1- 2002 062 083

## Beschreibung

Die Erfindung betrifft ein Videoendoskop, mit einem eine Längsachse aufweisenden langerstreckten Schaft und einem Handstück am proximalen Ende des Schafts, wobei an einem distalen Ende des Schafts ein Objektiv und proximalseitig des Objektivs ein elektronischer Bildaufnehmer angeordnet sind, wobei der Bildaufnehmer über eine sich längs des Schafts erstreckende elektrische Verbindung mit einem im Bereich des Handstücks angeordneten elektrischen Anschluss verbunden ist, und wobei der Bildaufnehmer und der Anschluss relativ zueinander um die Längsachse des Schafts verdrehbar sind.

Ein derartiges Videoendoskop ist aus DE 201 13 031 U1 bekannt.

Ein Videoendoskop ist ein Beobachtungsinstrument, das in der medizinischen Endoskopie verwendet wird. Durch die Verwendung eines Videoendoskops lassen sich im Rahmen der minimal-invasiven Chirurgie Körperregionen sowohl über natürliche Körperöffnungen als auch durch chirurgisch geschaffene künstliche Inzisionen erreichen. Bei Endoskopen allgemein unterscheidet man zwischen solchen, deren langerstreckter Schaft starr ist und solchen, deren langerstreckter Schaft flexibel ist. Die vorliegende Erfindung lässt sich sowohl bei starren Videoendoskopen als auch bei flexiblen Videoendoskopen einsetzen.

Ein Videoendoskop ist eine spezielle Gattung von Endoskopen, bei denen die Bilderfassung und Bildweiterleitung anstatt über ein Linsensystem oder ein geordnetes Glasfaserbündel über einen elektronischen Bildaufnehmer und elektrische Leitungen realisiert ist.

Aufgrund der heutigen Verfügbarkeit miniaturisierter elektronischer Bildaufnehmer, beispielsweise in CCD- oder CMOS-Technik, ist es möglich, den elektronischen Bildaufnehmer im distalen Ende des Schafts anzuordnen. Die Abbildung des beobachteten Objekts auf den Bildaufnehmer erfolgt dabei über ein distalseitig des elektronischen Bildaufnehmers angeordnetes Objektiv. Der elektronische Bildaufnehmer wandelt die empfangenen Photonen in elektrische Signale um, die über eine oder mehrere elektrische Leitungen, die sich vom Bildaufnehmer durch den Schaft hindurch bis zum Handstück des Videoendoskops erstrecken, nach proximal zu einem elektrischen Anschluss geleitet werden. Über den elektrischen Anschluss wird das Videoendoskop mittels eines Kabels mit einer Bildverarbeitungseinheit einschließlich Monitor verbunden, wobei auf dem Monitor das vom Bildaufnehmer erfasste Bild dargestellt wird. Die elektrische Verbindung, die sich durch den Schaft erstreckt, dient des Weiteren auch zur Ansteuerung und zur Spannungsversorgung des distalen Bildaufnehmers.

Bei Videoendoskopen mit distalem elektronischem Bildaufnehmer ist die Orientierung des Arztes hinsichtlich des auf dem Monitor dargestellten Videobildes erschwert, wenn das Videoendoskop während der Beobachtung eines Körperareals um seine Längsachse gedreht wird. Wenn der Bildaufnehmer nämlich relativ zum Schaft drehfest ist, wird der Bildaufnehmer bei einer Drehung des Videoendoskops um seine Längsachse mitgedreht, was zwangsläufig zur Drehung des Bildes auf dem Monitor führt.

Dieses Orientierungsproblem ist bei Videoendoskopen, deren Objektiv eine Geradeausblick- oder sogenannte 0°-Optik aufweist, durch Markierungen am Handgriff und im Monitorbild beherrschbar.

Gravierender stellt sich dieses Problem jedoch bei Videoendoskopen dar, deren Objektiv eine Schrägblickoptik, beispielsweise eine 30°-, 45°- oder 90°-Optik aufweist. Bei einer Drehung eines Videoendoskops mit Schrägblickoptik um seine Längsachse ändert sich sowohl die Blickrichtung als auch die Orientierung des Gesichtsfeldes in Bezug auf oben, unten, rechts und links. Selbst mit Markierungen am Handgriff des Videoendoskops und im Monitorbild kann sich der Arzt nicht sicher räumlich im beobachteten Körperareal orientieren.

Aus diesem Grund wurden Konzepte vorgeschlagen, mit denen eine Bildaufrichtung des Monitorbildes ermöglicht wird, so dass das Monitorbild stets eine definierte Orientierung in Bezug auf oben, unten, rechts und links besitzt.

Eines dieser Konzepte besteht darin, den Monitor selbst zu drehen. Eine Monitordrehung ist zwar möglich, setzt aber aufwändige elektromechanische Aufbauten voraus, die im Operationsumfeld kritisch zu betrachten sind. Außerdem ist das übliche 4 : 3 bzw. 16:9 (HDTV)-Bildgrößenverhältnis für eine mechanische oder auch eine analog-elektronische Drehung über die Strahlablenkung, wie in EP 7 12 289 A1 beschrieben, wenig geeignet.

Ein weiteres Konzept besteht darin, das auf dem Monitor dargestellte Bild mittels Bildverarbeitung zu drehen, wie beispielsweise in US 5,313,306 beschrieben ist. Die Bildaufrichtung mittels digitaler Bildverarbeitung hat jedoch den Nachteil, dass kein vollformatiges 4: 3-Bild ohne Informationsverlust gedreht werden kann. Nur der mittige Inkreis im 3 : 3-Quadrat kann ohne Informationsverlust gedreht werden. Die mit Videoendoskopen arbeitenden Ärzte sind jedoch mit formatfüllenden Bildern, insbesondere in der Laparoskopie (Bauchraumchirurgie), mit der entsprechenden formatfüllenden Bildinformation vertraut und wollen auf diese nicht verzichten.

Das dritte Konzept zur Bildaufrichtung, von dem die vorliegende Erfindung ausgeht, besteht darin, den elektronischen Bildaufnehmer verdrehbar auszugestalten. Bei einer Drehung des Videoendoskops um seine Längsachse kann der Bildaufnehmer zur Beibehaltung einer bestimmten Orientierung durch eine Drehung relativ zum Schaft und zum schaftfesten Objektiv raumfest gehalten werden. Der Bildaufnehmer ist dadurch von dem Objektiv entkoppelt, was insbesondere bei einem Schrägblickobjektiv von Vorteil ist.

Die Drehbarkeit des Bildaufnehmers relativ zum Schaft und damit auch zum proximalen elektrischen Anschluss stellt jedoch andere Herausforderungen an das Videoendoskop, insbesondere an die elektrische Verbindung zwischen dem Bildaufnehmer und dem proximalen elektrischen Anschluss.

Bei einer Verdrehung des Bildaufnehmers relativ zum Schaft wird zwangsläufig die zumindest eine elektrische Verbindung zwischen dem Bildaufnehmer und dem elektrischen Anschluss verdreht, wenn der elektrische Anschluss am proximalen Ende des Handstücks drehfest bezüglich des Schafts ist.

Bei dem o.g. Dokument DE 201 13 031 U1 ist die elektrische Verbindung zwischen dem Bildaufnehmer und dem elektrischen Anschluss durch ein mehradriges Kabel realisiert. Da bei einem Videoendoskop mit Schrägblickoptik die Verdrehbarkeit des Bildaufnehmers im und entgegen dem Uhrzeigersinn um 180° möglich sein sollte, wird das mehradrige Kabel entsprechend stark auf Torsion beansprucht. Nach einer gewissen Anzahl an Lastwechseln und/oder nach mehreren Sterilisationen in einem Autoklaven neigt das Kabel zum Brechen, wodurch die Lebensdauer des Videoendoskops nachteilig verringert ist.

Zur Lösung dieses Problems wird in DE 201 13 031 U1 vorgeschlagen, am proximalen Ende des Kabels einen Schleifkontakt vorzusehen, so dass das Kabel zusammen mit dem Bildaufnehmer torsionsfrei um seine Längsachse relativ zu dem elektrischen Anschluss gedreht werden kann. Diese Lösung hat jedoch den Nachteil, dass sie konstruktiv sehr aufwändig ist und darüber hinaus störanfällig ist.

Ein weiterer Nachteil der Ausgestaltung der elektrischen Verbindung mittels eines mehradrigen Kabels ist der hohe Aufwand bei der Herstellung des Videoendoskops, weil jede Ader einzeln mit dem Bildaufnehmer kontaktiert werden muss, was sich insbesondere bei miniaturisierter Ausgestaltung des Bildaufnehmers schwierig gestaltet.

Aus DE 10 2004 023 866 B3 ist ein Videoendoskop bekannt, das einen langerstreckten Schaft und ein darin distal angeordnetes Objektiv mit Videokamera aufweist. Die Videokamera weist Kamerakontakte auf, die über in dem Schaft verlaufende Leiter mit proximal in dem Schaft angeordneten Anschlusskontakten verbunden sind. Die Leiter sind als Leiterbahnen auf einer Leiterplatte ausgebildet. Die Leiterplatte ist flexibel ausgebildet, so dass sie in beliebiger Weise flexibel im Innenraum des Videoendoskopes verlegt und an den Enden mit den Kontaktierflächen in passende Lage zu den Kontakten gebracht werden kann. In einem weiteren in dem Dokument beschriebenen Ausführungsbeispiel ist die Leiterplatte aus starrem, gegebenenfalls auch dickerem Material ausgebildet. Die Leiterplatte kann dabei zur Verbesserung ihrer Drehsteifigkeit zu einem Dreiecksprofil geschlossen sein.

Aus dem Dokument US 6,488,631 B2 ist ein Ultraschallendoskop offenbart, bei dem die elektrische Verbindung zwischen distal angeordneten Ultraschallwandlern und proximalem Anschluss im Bereich einer distalen Biegestelle des Endoskops durch eine Vielzahl von flexiblen Leiterplatten realisiert sind, die aus einem Rohr geschnitten sind. Der elektronische Bildaufnehmer dieses Endoskops ist dagegen mittels eines mehradrigen Kabels elektrisch mit dem proximalen Anschluss verbunden.

Der Erfindung liegt die Aufgabe zugrunde, ein Videoendoskop der eingangs genannten Art dahingehend weiterzubilden, dass die elektrische Verbindung zwischen dem Bildaufnehmer und dem elektrischen Anschluss mit geringem Aufwand und daher in kostengünstiger Weise ausgestaltet ist, die darüber hinaus ermüdungssicher ist und die Standzeit des Videoendoskops entsprechend erhöht ist.

Erfindungsgemäß wird diese Aufgabe hinsichtlich des eingangs genannten Videoendoskops dadurch gelöst, dass die elektrische Verbindung zumindest eine in sich um ihre Längsrichtung verdrehbare flexible langerstreckte schmale Leiterplatte aufweist, die zumindest eine Leiterbahn aufweist, dass die elektrische Verbindung eine zweite in sich um ihre Längsrichtung verdrehbare flexible langerstreckte schmale Leiterplatte aufweist, die zumindest eine Leiterbahn aufweist, und dass die erste und die zweite Leiterplatte im Schaft übereinanderliegend und zueinander beweglich angeordnet sind, wobei die erste und die zweite Leiterplatte im Bereich ihres proximalen Endes über einen flexiblen Steg miteinander verbunden sind.

Bei dem erfindungsgemäßen Videoendoskop ist die elektrische Verbindung zwischen dem Bildaufnehmer und dem proximalen elektrischen Anschluss anstatt durch ein mehradriges Kabel durch zumindest eine an einer flexiblen länglichen schmalen Leiterplatte ausgebildete Leiterbahn realisiert. Durch die Flexibilität der zumindest einen Leiterplatte ist gewährleistet, dass sich die Leiterplatte bei einer Verdrehung des Bildaufnehmers relativ zum elektrischen Anschluss in sich verdrehen bzw. verwinden kann, ohne - auch nach einer Vielzahl von wechselseitigen Verdrehungen - zu brechen, und ohne dass die zumindest eine Leiterbahn bricht. Die zumindest eine Leiterbahn kann als sehr dünne Metallisierung auf der im übrigen isolierend ausgebildeten Leiterplatte ausgebildet werden. Diese zumindest eine Leiterbahn oder auch mehrere Leiterbahnen können in oder nahe der bei einer Verwindung der Leiterplatte neutralen Zone ausgebildet werden, in der sich eine Verwindung der Leiterplatte nicht oder nahezu nicht in einer Verwindung der Leiterbahn äußert, wodurch die Leiterbahn im wesentlichen nicht gestaucht oder gedehnt wird.

Die Verwendung zumindest einer Leiterplatte als elektrische Verbindung zwischen dem Bildaufnehmer und dem proximalen elektrischen Anschluss hat des Weiteren den Vorteil, dass auf einer Leiterplatte eine Mehrzahl von Leiterbahnen in Form von dünnen Metallisierungen, bspw. Kupfer, aufgebracht werden können, die für die Kontaktierung des Bildaufnehmers erforderlich sind, wie beispielsweise bei CCD-oder CMOS-Chips. Die Verwendung einer Mehrzahl von Kabeln oder eines mehradrigen Kabels entfällt somit vorteilhafterweise.

Die Leiterplatte hat des Weiteren den Vorteil, bei gleichzeitig hoher Flexibilität eine hohe mechanische Stabilität zu besitzen, ebenso eine Temperaturstabilität, die den Bedingungen in einem Autoklaven gut Stand halten kann. Die Realisierung der elektrischen Verbindung des Bildaufnehmers über zumindest eine Leiterplatte ist im Vergleich zu Kabelsystemen hinsichtlich der Gestehungskosten außerdem kostengünstig, weil die längliche flexible Leiterplatte und auch die später noch zu beschreibenden ggf. vorhandenen Leiterplattenteile aus einem einzigen planaren Material hergestellt werden können.

Auch der Aufwand beim Zusammenbauen des erfindungsgemäßen Videoendoskops ist durch die Verwendung zumindest einer Leiterplatte geringer, weil nicht mehrere Adern einzeln mit dem Bildaufnehmer kontaktiert werden müssen, sondern die Kontaktierung in einem einzigen Vorgang bspw. mit einem Balkenlötgerät erfolgen kann.

Die Leiterplatte ist eine erste Leiterplatte, und die elektrische Verbindung weist eine zweite in sich um ihre Längsrichtung verdrehbare flexible langerstreckte schmale Leiterplatte auf, die zumindest eine Leiterbahn aufweist.

Der Vorteil dieser Maßnahme besteht darin, dass zwei flexible längliche Leiterplatten zusammen mehr Leiterbahnen tragen können als eine Leiterplatte, und dabei beide Leiterplatten im Sinne einer höheren Flexibilität schmal ausgebildet werden können. Schmälere Leiterplatten haben wiederum den Vorteil, dass sie sich einfacher über die Länge im Rohr des Videoendoskops verdrehen lassen. Zwei Leiterplatten lassen sich aufgrund ihrer schmäleren Ausgestaltung auch einfacher in den Schaft des Videoendoskops integrieren, weil sie übereinanderliegend angeordnet werden können. Darüber hinaus ist zu berücksichtigen, dass elektrische Bildaufnehmer häufig zwei Reihen von seitlichen Anschlusspins aufweisen, so dass eine elektrische Verbindung über zwei Leiterplatten auch mechanisch besser an die Gegebenheiten des Bildaufnehmers angepasst ist.

Die erste und die zweite Leiterplatte sind im Schaft übereinanderliegend und zueinander beweglich angeordnet.

Diese Maßnahme hat den Vorteil, dass der Raum im Schaft des Videoendoskops durch die übereinanderliegende Anordnung, bei der die Leiterplatten demnach in zwei Ebenen angeordnet sind, besser genutzt werden kann. Durch die Beweglichkeit der beiden Leiterplatten zueinander wird die Flexibilität einer solchen Leiterplattenanordnung gegenüber einer einzelnen Leiterplatte nicht verringert.

Die erste und die zweite Leiterplatte sind im Bereich ihres proximalen Endes über einen flexiblen Steg miteinander verbunden.

Diese Maßnahme hat den Vorteil, dass die beiden Leiterplatten über den Steg, der dazu Leiterbahnen aufweist, miteinander elektrisch verbunden werden können, so dass nur eine der Leiterplatten mit dem Anschluss verbunden werden muss.

In einer bevorzugten Ausgestaltung weist die zumindest eine Leiterplatte eine Mehrzahl an Leiterbahnen auf, die nebeneinander und höchstens in zwei Ebenen der Leiterplatte angeordnet sind.

Diese Maßnahme hat den Vorteil, dass die zumindest eine Leiterplatte bei einer höchstens zweilagigen Ausgestaltung sehr dünn ausgebildet sein kann und die Leiterbahnen in der zentralen Zone angeordnet werden können, in der sie durch die Biegebeanspruchung der zumindest einen Leiterplatte weder wesentlich gedehnt noch wesentlich gestaucht werden. Da die Leiterbahnen linienförmig auf die Leiterplatte aufgebracht werden können, können auf einer solchen Leiterplatte eine Mehrzahl von Leiterbahnen nebeneinander angeordnet werden.

In einer weiteren bevorzugten Ausgestaltung weist die zweite Leiterplatte eine Mehrzahl an Leiterbahnen auf, die nebeneinander und in höchstens zwei Ebenen der zweiten Leiterplatte angeordnet sind.

Auch hier ist wiederum von Vorteil, dass einlagige bzw. höchstens zweilagige Leiterplatten eine optimale Flexibilität und dennoch ausreichende Stabilität besitzen.

Der flexible Steg befindet sich vorzugsweise im Handstück des Videoendoskops und ist dort nicht in dem Maße einer Verdrehbeanspruchung unterworfen wie die Leiterplatten, die sich durch den Schaft hindurch erstrecken.

In einer weiteren bevorzugten Ausgestaltung schließt sich an die zumindest eine Leiterplatte proximalseitig zumindest ein proximales, vorzugsweise starres, Leiterplattenteil an, das elektrische Bauteile trägt.

Hierbei ist von Vorteil, dass weitere elektronische Bauelemente zur Bildaufnehmersteuerung, wie beispielsweise für die Identifikation des Bildaufnehmers, Längenlaufzeitkompensation des Videoendoskops, Verstärkungsanpassung, in die elektrische Verbindung integriert werden können. Eine starre Ausgestaltung des Leiterplattenteils hat den Vorteil, dass sie sich leichter mit den Bauteilen konfektionieren lässt.

Dabei ist es weiterhin bevorzugt, wenn das proximale Platinenteil mit einem weiteren proximalen, vorzugsweise starren, Leiterplattenteil, das elektrische Bauteile trägt, über einen flexiblen Steg verbunden ist.

Hierbei ist von Vorteil, dass die Anzahl an elektrischen Bauteilen, die auf der Leiterplatte proximal angeordnet werden können, erhöht werden kann, wobei die beiden proximalen Leiterplattenteile vorteilhafterweise über den flexiblen Steg im Handstück elektrisch miteinander verbunden sein können.

In einer vorteilhaft platzsparenden Anordnung sind die beiden proximalen Leiterplattenteile im Handgriff übereinanderliegend angeordnet.

In einer weiteren bevorzugten Ausgestaltung weist das zumindest eine proximale Leiterplattenteil den elektrischen Anschluss auf.

Hierbei ist von Vorteil, dass der elektrische Anschluss bereits bei der Herstellung der gesamten elektrischen Verbindung aus Leiterplatte(n) und proximalen Plattenteil(en) in diese integriert werden kann, so dass die gesamte elektrische Verbindung als Baueinheit vorkonfektioniert in das Handstück und den Schaft eingebaut werden kann, was den Herstellungsaufwand des Videoendoskops reduziert.

In einer weiteren bevorzugten Ausgestaltung weist die zumindest eine Leiterplatte an ihrem distalen Ende ein distales, vorzugsweise starres, Leiterplattenteil zur Kontaktierung mit dem Bildaufnehmer auf.

Das distale Leiterplattenteil dient als Schnittstelle zum Bildaufnehmer, was den Vorteil einer einfach zu bewekstelligenden Kontaktierung hat.

Das oder die starren Leiterplattenteile können, wie in einer weiteren bevorzugten Ausgestaltung vorgesehen ist, einstückig mit der flexiblen Leiterplatte aus einem planaren Trägermaterial gefertigt sein. Damit das oder die distalen Leiterplattenteile starr sind, können sie aus demselben Trägermaterial wie die Leiterplatte bzw. Leiterplatten selbst gefertigt sein, das dann im Bereich der distalen Leiterplattenteile verstärkt ist.

Im Fall von zwei Leiterplatten mit jeweils einem distalen starren Leiterplattenteil können die beiden distalen Leiterplattenteile mechanisch und elektrisch verbunden sein, beispielsweise durch zwei durchgesteckte verlötete Drähte, um die mechanische Festigkeit im Bereich der Kontaktierung mit dem Bildaufnehmer zu erhöhen.

In einer weiteren bevorzugten Ausgestaltung ist das distale Leiterplattenteil dicker und/oder breiter als die sich an das distale Leiterplattenteil anschließende Leiterplatte.

Die Flexibilität der elektrischen Verbindung muss im Wesentlichen sich durch den Schaft erstreckenden Abschnitt, also im Bereich der flexiblen Leiterplatte gewährleistet sein. Das distale Leiterplattenteil ist dagegen vorzugsweise starr und kann daher auch dicker und/oder breiter als der sich an das distale Leiterplattenteil anschließende Teil der Leiterplatte ausgebildet sein, wodurch die zusätzliche Belegung des distalen Leiterplattenteils mit elektrischen Bauteilen vorteilhafterweise ermöglicht wird.

Dabei ist es weiterhin bevorzugt, wenn die Breite des distalen Leiterplattenteils und/oder die Breite der zumindest einen Leiterplatte in ihrem sich durch den Schaft erstreckenden Abschnitt höchstens so groß ist wie die Breite des Bildaufnehmers.

Hierbei ist von Vorteil, dass sich die zumindest eine Leiterplatte mit ihrem starren distalen Leiterplattenteil bzw. die zwei Leiterplatten mit ihrem jeweiligen distalen starren Leiterplattenteil zur Montage einfach von proximal nach distal durch den Schaft des Videoendoskops schieben lassen, wonach sie mit dem Bildaufnehmer kontaktiert werden können.

In einer weiteren bevorzugten Ausgestaltung nimmt die Flexibilität der zumindest einen Leiterplatte von proximal nach distal bis zu dem distalen Leiterplattenteil kontinuierlich oder in Stufen ab.

Bei dieser Ausgestaltung wird die zumindest eine Leiterplatte von distal nach proximal zunehmend flexibler, was den Vorteil hat, dass die Belastung bei der Verdrehung der zumindest einen Leiterplatte nicht an der Übergangsstelle zu dem starren distalen Leiterplattenteil wirkt, sondern sich über einen länglichen Bereich nach proximal verteilt.

In einer weiteren bevorzugten Ausgestaltung ist die zumindest eine Leiterplatte einstückig aus einem planaren Trägermaterial gefertigt.

Hierbei ist von Vorteil, dass die Leiterplatte kostengünstig hergestellt werden kann.

Ebenso bevorzugt ist es im Zusammenhang mit einer der o.g. Ausgestaltungen, wonach zwei Leiterplatten vorgesehen sind, die über einen flexiblen Steg miteinander verbunden sind, wenn die erste und die zweite Leiterplatte und der sie verbindende Steg einstückig aus einem planaren Trägermaterial gefertigt sind.

Ebenso ist es bevorzugt, im Zusammenhang mit einer der o.g. Ausgestaltungen, wonach die zumindest eine Leiterplatte ein proximales Leiterplattenteil aufweist, wenn die zumindest eine Leiterplatte und das zumindest eine proximale Leiterplattenteil einstückig aus einem planaren Trägermaterial gefertigt sind.

Im Fall, dass wie in einer der o.a. Ausgestaltungen die zumindest eine Leiterplatte ein distales Leiterplattenteil aufweist, ist es bevorzugt, wenn die zumindest eine Leiterplatte und das zumindest eine distale Leiterplattenteil einstückig aus einem planaren Trägermaterial gefertigt sind.

In einer weiteren bevorzugten Ausgestaltung ist das zuvor genannte Trägermaterial ein polymerer Kunststoff, insbesondere Polyimid.

Ein polymerer Kunststoff, insbesondere Polyimid, hat den Vorteil einer hohen Flexibilität bzw. In-Sich-Verdrehbarkeit, ebenso wie guter Isolationseigenschaften und einer Gewichtsersparnis. Polyimid hat insbesondere den Vorteil einer sehr hohen thermischen Stabilität bei Temperaturen bis über 200°C, was für eine Sterilisierung des Videoendoskops in einem Autoklaven sehr vorteilhaft ist.

Alternativ zur insgesamt einstückigen Bauweise der zumindest einen Leiterplatte und/oder der Leiterplattenteile ist es vorzugsweise vorgesehen, dass die zumindest eine Leiterplatte aus einer Mehrzahl von Leiterplattenabschnitten aufgebaut ist, die unterschiedliche Flexibilitäten aufweisen.

Dazu kann die Leiterplatte abschnittsweise aus verschieden flexiblen Trägermaterialien aufgebaut sein. Ebenso können insbesondere das o.g. distale Leiterplattenteil und das proximale Leiterplattenteil als starre Platten ausgebildet sein, die stabiler als die Leiterplatte sind und sich somit besser für die Bestückung speziell für elektrische Bauteile eignen. Insbesondere lässt sich das zumindest eine proximale Leiterplattenteil bei einer starren Ausgestaltung insbesondere im Handstück leichter einbauen. Die Kontaktierung von elektrischen Bauteilen ebenso wie die Kontaktierung des Bildaufnehmers am distalen Leiterplattenteil sowie des elektrischen Anschlusses am proximalen Leiterplattenteil gestalten sich über starre Leiterplattenteile einfacher.

In einer weiteren bevorzugten Ausgestaltung ist die zumindest eine Leiterplatte in ihrem flexiblen Bereich aus mehreren, vorzugsweise zwei Leiterplattenlagen aufgebaut.

Während einlagige Leiterplatten den Vorteil einer sehr hohen Flexibilität besitzen, hat die Mehrlagigkeit der Leiterplatten, die auch abschnittsweise vorgesehen sein kann, den Vorteil, dass komplexe Schaltungsaufbauten auf engstem Raum realisiert werden können.

Weitere Vorteile und Merkmale ergeben sich aus der nachfolgenden Beschreibung und der beigefügten Zeichnung.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hiernach näher beschrieben. Es zeigen:
- Figur 1: eine perspektivische Darstellung eines Videoendoskops gemäß einem Beispiel, wobei Teile des Videoendoskops weggelassen wurden, und wobei das Videoendoskop teilweise im Längsschnitt dargestellt ist;
- Figur 1A: einen vergrößerten Ausschnitt eines Details des Videoendoskops in Figur 1;
- Figur 2: einen Ausschnitt des Videoendoskops in Figur 1 in vergrößertem Maßstab und im Längsschnitt;
- Figur 3: eine zu Figur 1 vergleichbare Darstellung des Videoendoskops in Figur 1 in einem gegenüber Figur 1 veränderten Betriebszustand;
- Figur 4: eine zu Figur 2 vergleichbare Darstellung des Videoendoskops in dem in Figur 3 gezeigten Betriebszustand;
- Figur 5: eine perspektivische Darstellung eines Videoendoskops gemäß einem Ausführungsbeispiel, wobei Teile des Videoendoskops weggelassen sind und das Videoendoskop teilweise im Längsschnitt dargestellt ist;
- Figur 6: einen Ausschnitt des Videoendoskops in Figur 5 im vergrößerten Maßstab und im Längsschnitt;
- Figur 7: das Videoendoskop in Figur 5 in einer zu Figur 5 vergleichbaren Dar- stellung, jedoch in einem gegenüber Figur 5 geänderten Betriebszu- stand;
- Figur 8: das Videoendoskop in Figur 5 in einer zu Figur 6 vergleichbaren Dar- stellung in dem Betriebszustand des Videoendoskops gemäß Figur 7; und
- Figur 9: ein weiteres Ausführungsbeispiel einer elektrischen Verbindung für das Videoendoskop in Figur 1 oder in Figur 5.

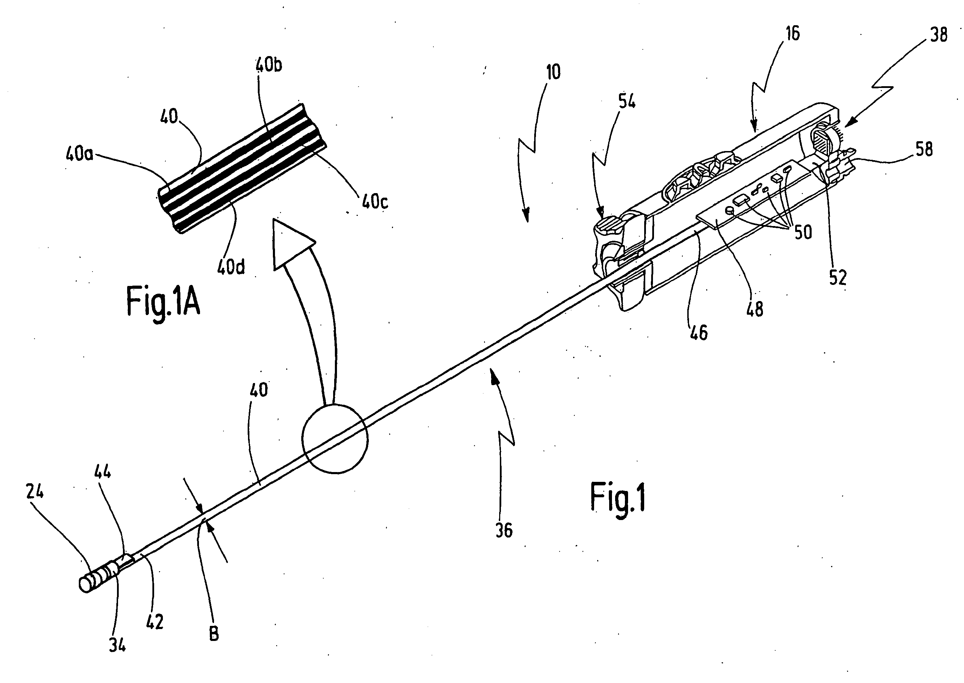

In Figuren 1 bis 4 ist ein Beispiel eines mit dem allgemeinen Bezugszeichen 10 versehenen Videoendoskops dargestellt. Ein Detail des Videoendoskops 10 ist in Figur 1A dargestellt.

Das Videoendoskop 10 wird beispielsweise im Rahmen der minimal-invasiven Chirurgie zur Beobachtung eines Körperareals im Inneren des Körpers verwendet.

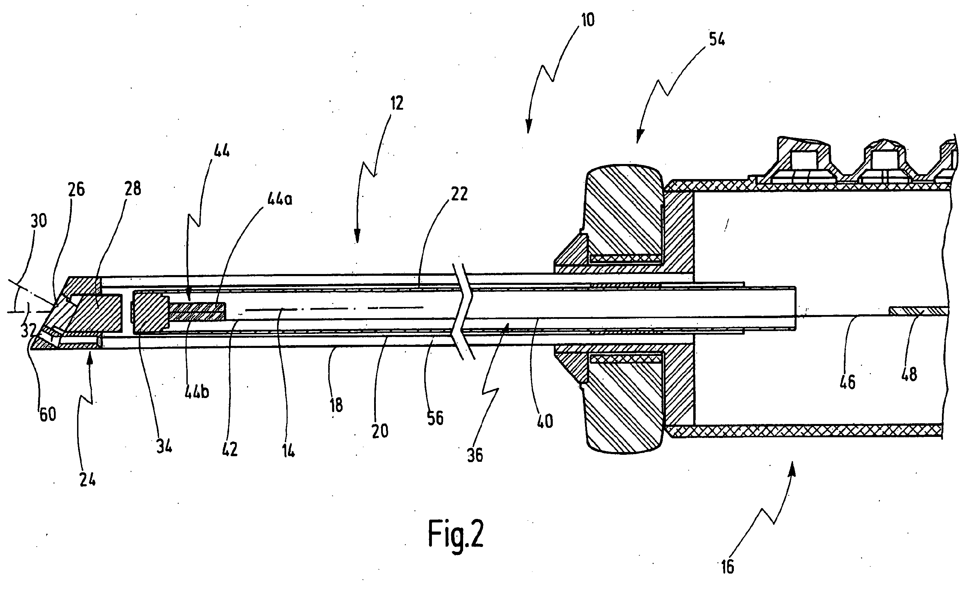

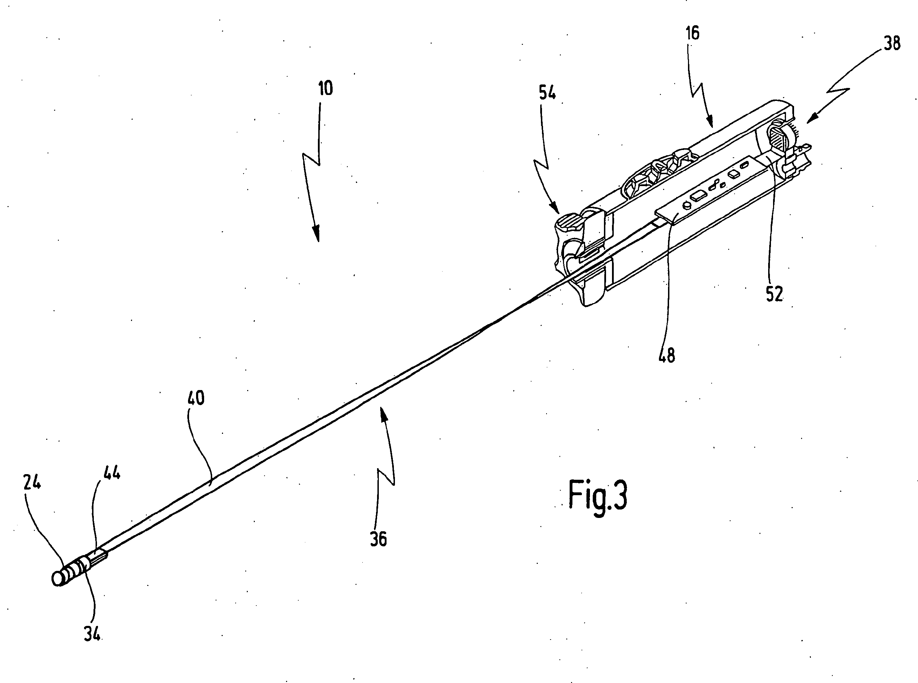

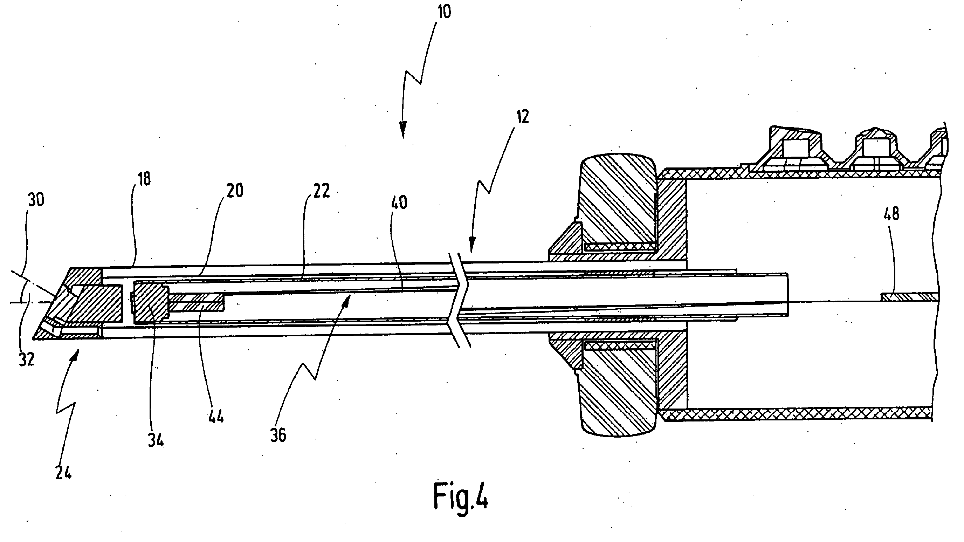

Das Videoendoskop 10 weist gemäß Figuren 2 und 4 einen langerstreckten Schaft 12 auf, der in Figuren 1 und 3 aus Gründen der Übersichtlichkeit weggelassen wurde. Der Schaft 12 weist eine Längsachse 14 auf. Am proximalen Ende des Schafts 12 weist das Videoendoskop 10 ein Handstück 16 auf, mit dem der Schaft 12 verbunden ist.

In dem gezeigten Ausführungsbeispiel ist der Schaft 12 aus mehreren ineinander geschobenen Rohren aufgebaut, und zwar einem äußeren Rohr 18, einem darin angeordneten mittleren Rohr 20 und einem inneren Rohr 22. Der Aufbau des Schafts 12 aus einer Mehrzahl von Rohren, wie hier den Rohren 18, 20 und 22 ist optional, und der Schaft 12 kann auch aus einem einzigen, aus zwei oder mehr als drei Rohren aufgebaut sein. Das innere Rohr 22 ist relativ zu den anderen Beiden Rohren 18, 20 um die Längsachse 14 verdrehbar.

Am distalen Ende des Schafts 12 ist ein Objektiv 24 angeordnet, das beispielhaft zwei optische Elemente 26 und 28 aufweist. Das Objektiv 24 ist ein Schrägblickobjektiv, d.h. eine Blickrichtung 30 des Objektivs 24 schließt mit der Längsachse 14 des Schafts 12 einen Winkel 32 von ≠ 0° ein, im gezeigten Ausführungsbeispiel beträgt der Winkel 32 30°. Das Objektiv ist drehfest mit dem mittleren und dem äußeren Rohr 18, 20 des Schafts 12 verbunden.

Proximalseitig des Objektivs 24, jedoch immer noch am distalen Ende des Schafts 12, ist weiterhin ein elektronischer Bildaufnehmer 34 angeordnet. Der elektronische Bildaufnehmer 34 ist in dem gezeigten Ausführungsbeispiel mit dem distalen Ende des inneren Rohrs 22 fest verbunden.

Zwischen dem Objektiv 24 und dem Bildaufnehmer 34 können noch weitere optische Elemente, wie bspw. ein oder mehrere Filter, angeordnet sein.

Der elektronische Bildaufnehmer 34 ist beispielsweise in Form eines CCD- oder CMOS-Chips ausgebildet.

Das Objektiv 24 bildet das beobachtete Körperareal auf den Bildaufnehmer 34 ab. Der Bildaufnehmer 34 wandelt die empfangenen optischen Signale in elektrische Signale um.

Der elektronische Bildaufnehmer 34 ist über eine elektrische Verbindung 36 mit einem elektrischen Anschluss 38, der hier am proximalen Ende des Handstücks 16 angeordnet ist, verbunden. Der elektrische Anschluss 38 ist als Steckbuchse ausgebildet.

Die elektrische Verbindung 36 wird durch zumindest eine, in dem Beispiel gemäß Figuren 1 bis 4 genau eine flexible längliche schmale Leiterplatte 40 bewerkstelligt, die in sich um ihre Längsrichtung, die der Richtung der Längsachse 14 entspricht, verdrehbar ist.

Wie in dem vergrößerten Ausschnitt in Figur 1A dargestellt ist, trägt die Leiterplatte 40 eine Mehrzahl an Leiterbahnen, hier beispielhaft vier Leiterbahnen 40a, 40b auf einer ihrer Oberflächen, die beispielsweise als dünne Kupferlinien ausgebildet sind. Auf der gegenüberliegenden Seite kann die Leiterplatte 40 weitere Leiterbahnen tragen.

An einem distalen Ende 42 der Leiterplatte 40 schließt sich distalseitig ein distales Leiterplattenteil 44 an, das starr ist oder zumindest steifer als die flexible Leiterplatte 40. Das distale Leiterplattenteil 44 dient zur Kontaktierung mit dem elektronischen Bildaufnehmer 34. Wie aus Figur 1 hervorgeht, ist die Breite B der Leiterplatte 40 sowie des distalen Leiterplattenteils 44 nicht größer als die entsprechende Breite des Bildaufnehmers 34. Das distale Leiterplattenteil 44 ist, wie aus Figur 2 hervorgeht, dicker bzw. stärker als die Leiterplatte 40, die zur Erzielung einer hohen Flexibilität möglichst dünn ausgebildet ist.

Die Leiterplatte 40 ist hier einlagig aus einem planaren Trägermaterial, insbesondere Polyimid, aufgebaut, wobei die Leiterbahnen 40a, bis 40b auf das Trägermaterial beispielsweise aufgedampft sind.

Die Stärke oder Dicke der Leiterplatte 40 beträgt vorzugsweise 0,02 bis 0,3 mm.

Das distale Leiterplattenteil 44 trägt elektrische Bauteile, beispielsweise eine Verstärkerschaltung zur (Vor-) Verstärkung der vom Bildaufnehmer 34 erzeugten elektrischen Videosignale.

Die Leiterplatte 40 erstreckt sich durch den Schaft 12 hindurch bis in das Handstück 16, wobei sich an ein proximales Ende 46 der Leiterplatte 40 ein proximales Leiterplattenteil 48 anschließt.

Das proximale Leiterplattenteil 48 trägt weitere elektrische Bauteile 50 zur Steuerung des Bildaufnehmers 34.

Das proximale Leiterplattenteil 48 ist wiederum steifer als die Leiterplatte 40 und kann insbesondere auch starr sein, was die Aufbringung der elektrischen Bauteile 50 vereinfacht.

Das proximale Leiterplattenteil 48 weist proximalseitig den elektrischen Anschluss 38 auf, der hier als Steckerkontaktierung ausgebildet ist. Der elektrische Anschluss 38 ist mit dem proximalen Leiterplattenteil 48 über einen flexiblen Steg 52 verbunden.

Die Leiterplatte 40, das distale Leiterplattenteil 44 und das proximale Leiterplattenteil 48 nebst dem Steg 52 können insgesamt einstückig aus dem gleichen Trägermaterial gefertigt sein, wobei die unterschiedlichen Flexibilitätsgrade zwischen der Leiterplatte 40, dem distalen Leiterplattenteil 44 und dem proximalen Leiterplattenteil 48 durch entsprechende Verstärkung des Trägermaterials im Bereich des distalen Leiterplattenteils 44 und des proximalen Leiterplattenteils 48 erhalten werden können.

Die Anordnung aus Leiterplatte 40, distalem Leiterplattenteil 44 und proximalem Leiterplattenteil 48 kann jedoch auch als Hybridanordnung ausgebildet sein, d.h. die distalen und proximalen Leiterplattenteile 44 und 48 können aus einem Trägermaterial, insbesondere einem steifen Trägermaterial gefertigt sein, während die Leiterplatte 40 aus einem besonders flexiblen Trägermaterial, insbesondere einem polymeren Kunststoff, insbesondere Polyimid, gefertigt sein kann, wobei die einzelnen Plattenteile dann auf geeignete Weise, beispielsweise durch Kleben, miteinander verbunden werden. Der Kunststoff kann auch duroplastisch sein.

Die Leiterplatte 40 ist vorzugsweise einlagig, und die Leiterbahnen 40a bis 40d sind in höchstens zwei Ebenen der Leiterplatte angeordnet, beispielsweise auf den beiden gegenüberliegenden Breitseiten der Leiterplatte 40.

Die Leiterplatte 40 kann jedoch auch mehrlagig, vorzugsweise aus zwei Leiterplattenlagen aufgebaut sein, die miteinander fest verbunden werden. Im Sinne einer möglichst hohen Flexibilität der Leiterplatte 40 ist jedoch darauf zu achten, dass die Dicke bzw. Stärke der Leiterplatte 40 möglichst gering gehalten wird.

Es kann auch vorgesehen sein, die Leiterplatte 40 zwischen ihrem proximalen Ende 46 und ihrem distalen Ende 42 mit unterschiedlichen Flexibilitätsgraden auszustatten, wobei die Flexibilität dann vom proximalen Ende 46 zum distalen Ende 42 hin abnimmt, d.h. im Bereich des distalen Endes 42 ist die Leiterplatte 40 steifer als im Bereich des proximalen Endes 46.

Wie aus Figur 2 hervorgeht, ist das distale Leiterplattenteil 44 aus zwei Leiterplattenteillagen 44a und 44b aufgebaut.

Der elektronische Bildaufnehmer 34 ist des Weiteren relativ zu dem elektrischen Anschluss 38 und zu dem Objektiv 24 um die Längsachse 14 des Schafts 12 verdrehbar. Zur Verdrehbarkeit ist hier ein Stellglied 54 vorgesehen, das auf das innere Rohr 22 wirkt und dieses um die Längsachse 14 des Schafts dreht, wodurch der Bildaufnehmer 34, der mit dem inneren Rohr 22 fest verbunden ist, ebenfalls um die Längsachse 14 gedreht wird. Durch die Drehung des Bildaufnehmers 34 ist es möglich, das auf dem Monitor (nicht dargestellt) dargestellte Videobild stets in einer gewünschten Orientierung bezüglich oben, unten, rechts und links zu halten, d.h. das Videobild in gewünschter Weise auszurichten. Der Bildaufnehmer 34 ist dabei in beiden Drehrichtungen (Uhrzeigersinn und Gegenuhrzeigersinn) um die Längsachse 14 um vorzugsweise 180° verdrehbar.

Eine Verdrehung des Bildaufnehmers 34 relativ zu dem elektrischen Anschluss 38 bedingt nun eine Verdrehung der Leiterplatte 40 in sich, wie in Figuren 3 und 4 dargestellt ist.

Diese Verdrehung der Leiterplatte 40 wird dadurch ermöglicht, dass die Leiterplatte 40 in sich verdrehbar flexibel ausgebildet ist. In Figur 3 ist der Zustand dargestellt, in dem der Bildaufnehmer 34 gegenüber der Lage in Figur 1 um 180° um die Längsachse 14 verdreht ist. Das proximale Leiterplattenteil 48 hat sich dabei nicht oder im Wesentlichen nicht verdreht, während das distale Leiterplattenteil 44 sich mit dem Bildaufnehmer 34 ebenfalls um 180° verdreht hat. Die Leiterbahnen 40a bis 40d werden aufgrund der schmalen Breite B der Leiterplatte 40 nicht oder kaum auf Stauchung und Dehnung beansprucht, wodurch ein Bruch der Leiterbahnen 40a bis 40d auch im Langzeitgebrauch des Videoendoskops 10 nicht zu befürchten ist.

Das Videoendoskop 10 weist zwischen dem äußeren Rohr 18 und dem mittleren Rohr 20 einen Kanal 56 auf, in dem Lichtleitfasern für das Beleuchtungslicht verlaufen, wobei am proximalen Ende ein Anschluss 58 für ein nicht dargestelltes Lichtleitkabel vorgesehen ist, und wobei das Licht am distalen Ende des Schafts 12 an einem Lichtaustritt 60 austritt.

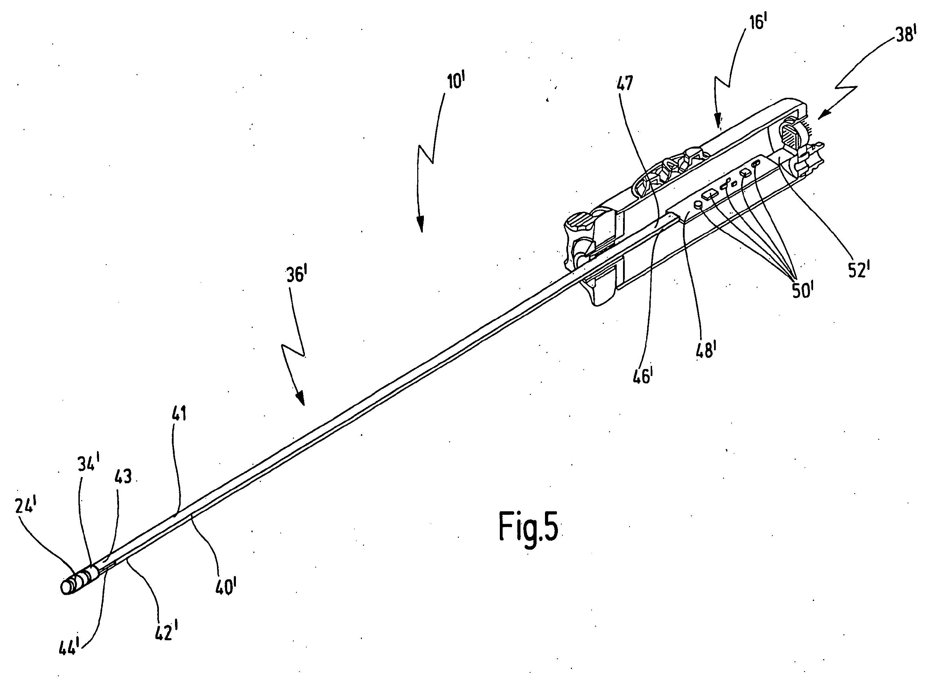

In Figuren 5 bis 8 ist ein erstes Ausführungsbeispiel eines Videoendoskops 10' dargestellt, bei dem in bezug auf das Videoendoskop 10 gleiche oder vergleichbare Teile mit den gleichen Bezugszeichen, ergänzt durch einen ', versehen sind. Nachfolgend werden nur die Unterschiede zu dem Videoendoskop 10 beschrieben.

Bei dem Videoendoskop 10' ist die elektrische Verbindung 36' zwischen dem Bildaufnehmer 34' und dem elektrischen Anschluss 38' durch zwei Leiterplatten 40' und 41 realisiert, die in dem Schaft 12' entlang der Längsachse 14' übereinander liegend angeordnet sind.

An das distale Ende 42' bzw. 43' der Leiterplatten 40' und 41 schließt sich wieder das distale Leiterplattenteil 44' an.

Durch die Verwendung von zwei Leiterplatten 40' und 41 lässt sich ohne Verbreiterung der einzelnen Leiterplatten 40' oder 41'die Anzahl an Leiterbahnen für die elektrische Verbindung zwischen dem Bildaufnehmer 34' und dem Anschluss 38' erhöhen, ohne dass die Dicke bzw. Stärke und damit die Steifigkeit der Leiterplatten 40' und 41 erhöht wird. Die Leiterplatte 41 ist ebenso wie die Leiterplatte 40' flexibel und in sich um ihre Längsrichtung verdrehbar.

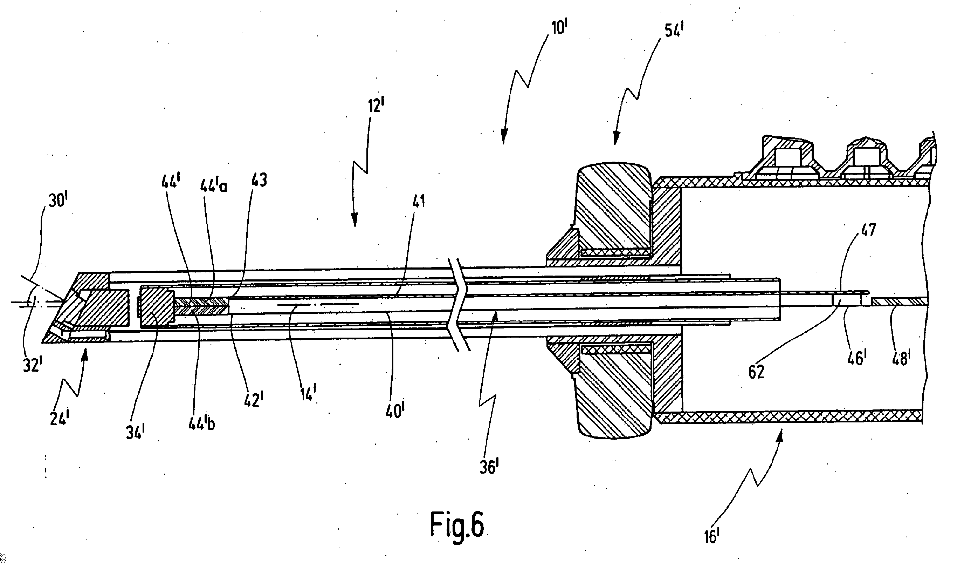

Die Leiterplatte 41 und die Leiterplatte 40' sind, wie aus Figur 6 hervorgeht, voneinander beabstandet und somit zueinander beweglich, wodurch die Gesamtflexibilität der beiden Leiterplatten 40' und 41 gegenüber nur einer Leiterplatte nicht oder nicht wesentlich verringert ist.

An ihrem proximalen Ende 46' bzw. 47 sind die Leiterplatten 41 und 40' über einen flexiblen Steg 62 miteinander verbunden, wobei der Steg 62 außerdem eine elektrische Verbindung zwischen den Leiterplatten 40' und 41 herstellt. Das proximale Leiterplattenteil 48' muss somit nur mit der Leiterplatte 40' elektrisch verbunden sein.

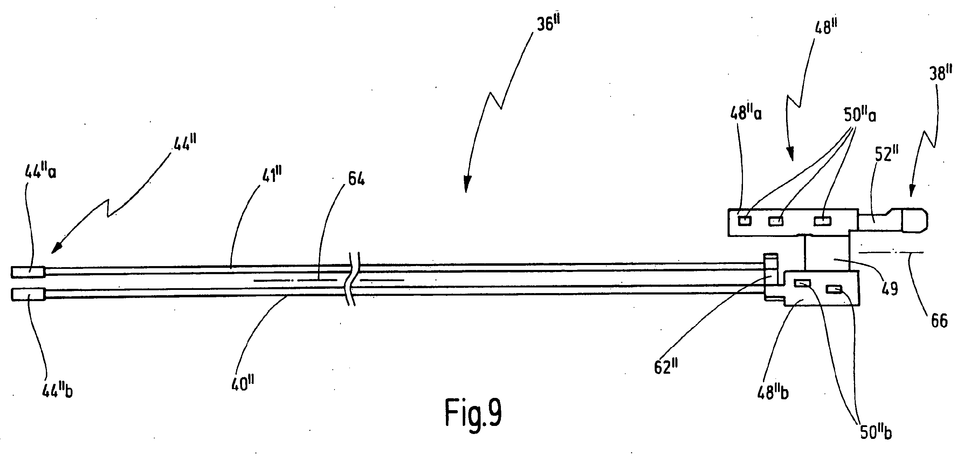

Vor dem Einbau der Leiterplatten 40' und 41 sind diese vorzugsweise auseinandergeklappt (vgl. auch Fig. 9), so dass sie in einer Ebene liegen. Dabei ist die Leiterplattenteillage 44'a mit der Leiterplatte 41 und die Leiterplattenteillage 44'b des distalen Leiterplattenteils 44' mit der Leiterplatte 40' verbunden. Zum Einführen der Leiterplatten 40' und 41 werden diese dann über den Steg 62 in übereinander liegende Anordnung gebogen, wie in Figur 6 dargestellt ist, wobei die Leiterplattenteile 44'a und 44'b beim Einführen gegeneinander verschoben sind, damit sie leichter durch den Schaft (inneres Rohr 22) einschiebbar sind.

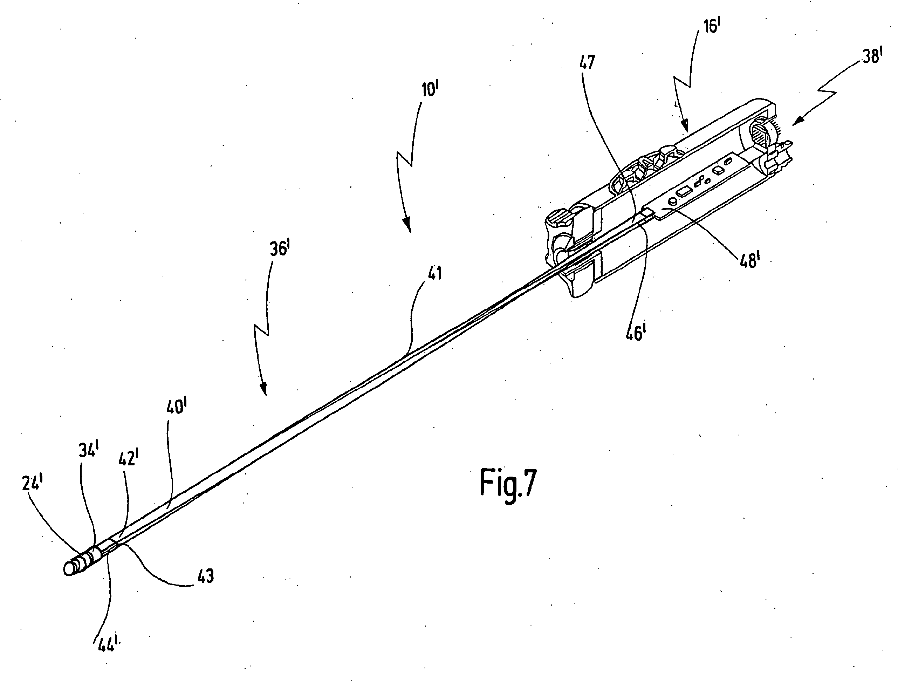

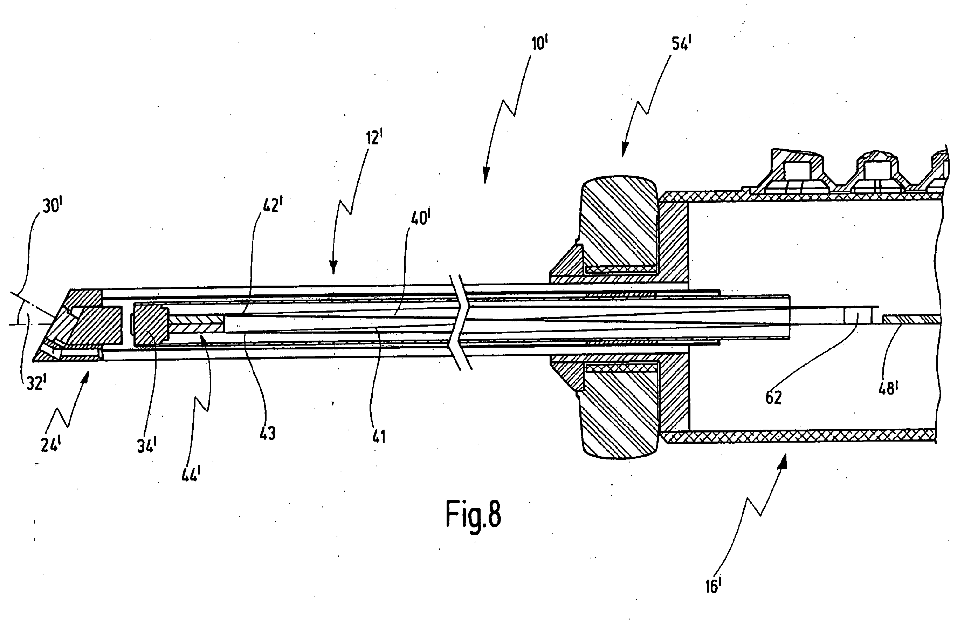

Figur 7 zeigt das Videoendoskop 10' in einem Zustand, in dem der Bildaufnehmer 34' um 180° aus seiner Lage in Figur 6 verdreht wurde. Wie aus Figuren 7 und 8 hervorgeht, gewährleistet die übereinander liegende beabstandete Anordnung der Leiterplatten 40' und 41, dass diese sich relativ zueinander bewegen und jeweils in sich verdrehen können.

Bei der Ausgestaltung der elektrischen Verbindung 36' mittels zwei Leiterplatten 40' und 41 können diese zusammen mit den Leiterplattenteilen 44' und 48' und dem Steg 62 aus demselben planaren Trägermaterial in einstückiger Weise gefertigt sein, oder sie können in Hybridbauweise aus unterschiedlichen Trägermaterialien in Bezug auf die Leiterplattenteile 44' und 48', die Leiterplatten 40', 41 ausgebildet sein.

Figur 9 zeigt eine weitere Abwandlung einer elektrischen Verbindung 36", die bei dem Videoendoskop 10 oder dem Videoendoskop 10' an Stelle der dortigen elektrischen Verbindungen 36 und 36' verwendet werden kann.

Auch hier wurden wiederum vergleichbare oder gleiche Teile wie in dem Ausführungsbeispiel gemäß Figuren 1 bis 4 bzw. 5 bis 8 mit den gleichen Bezugszeichen, ergänzt durch ", versehen.

Die elektrische Verbindung 36" weist hier zwei Leiterplatten 40" und 41" auf, wobei die Leiterplatte 40" an ihrem distalen Ende ein distales Leiterplattenteil 44b" und die Leiterplatte 41" an ihrem distalen Ende ein distales Leiterplattenteil 44a" aufweist. Insoweit entspricht die Anordnung der Ausgestaltung in Figuren 5 bis 8, wobei die Leiterplatten 40" und 41" noch nebeneinander liegend angeordnet sind. An ihrem proximalen Ende sind die Leiterplatten 40" und 41" über den flexiblen Steg 62" miteinander verbunden. Im Einbauzustand in dem Videoendoskop 10 oder 10' wird die Leiterplatte 41" mit ihrem distalen Leiterplattenteil 44a" um eine Spiegelachse 64 um 180° aus der Zeichenebene heraus auf die Leiterplatte 40" umgeklappt, so dass die daraus resultierende Anordnung der Anordnung der Leiterplatten 40' und 41 gemäß Figuren 5 bis 8 entspricht.

Die elektrische Verbindung 36" unterscheidet sich von den vorherigen elektrischen Verbindungen 36 und 36' dadurch, dass das proximale Leiterplattenteil 48" zweiteilig aufgebaut ist, und zwar aus einem ersten Leiterplattenteil 48a" und einem zweiten Leiterplattenteil 48b". Die Leiterplattenteile 48a" und 48b" sind über einen flexiblen Steg 49 verbunden, der die Leiterplattenteil 48a" und 48b" nicht nur mechanisch, sondern auch elektrisch miteinander verbindet.

An dem Leiterplattenteil 48a" ist über den flexiblen Steg 52" der elektrische Anschluss 38" vorgesehen.

In der Einbaulage der elektrischen Verbindung 36" in dem Videoendoskop 10 oder dem Videoendoskop 10' sind die Leiterplattenteile 48a" und 48b" um eine Achse 66 aufeinandergeklappt, d.h. sie liegen dann im Handstück 16 oder 16' übereinander. Beide Leiterplattenteile 48a" und 48b" tragen dabei die elektrischen Bauteile 50a" und 50b".

## Patentansprüche

1. Videoendoskop, mit einem eine Längsachse (14; 14') aufweisenden langerstreckten Schaft (12; 12') und einem Handstück (16; 16') am proximalen Ende des Schafts (12; 12'), wobei an einem distalen Ende des Schafts (12; 12') ein Objektiv (24; 24') und proximalseitig des Objektivs (24; 24') ein elektronischer Bildaufnehmer (34; 34') angeordnet sind, wobei der Bildaufnehmer (34; 34') über eine sich längs des Schafts (12; 12') erstreckende elektrische Verbindung (36; 36'; 36") mit einem am Handstück (16; 16') angeordneten elektrischen Anschluss (38; 38') verbunden ist, und wobei der Bildaufnehmer (34; 34') und der Anschluss (38; 38') relativ zueinander um die Längsachse (14; 14') des Schafts (12; 12') verdrehbar sind, **dadurch gekennzeichnet, dass** die elektrische Verbindung (36; 36'; 36") eine in sich um ihre Längsrichtung verdrehbare flexible langerstreckte schmale Leiterplatte (40; 40'; 40") aufweist, die zumindest eine Leiterbahn (40a-d) aufweist, dass die elektrische Verbindung (36'; 36") eine zweite in sich um ihre Längsrichtung verdrehbare flexible langerstreckte schmale Leiterplatte (41; 41") aufweist, die zumindest eine Leiterbahn (40a-d) aufweist, und dass die erste und die zweite Leiterplatte (40', 41; 40", 41") im Schaft (12') übereinanderliegend und zueinander beweglich angeordnet sind, wobei die erste und die zweite Leiterplatte (40', 41; 40", 41") im Bereich ihres proximalen Endes über einen flexiblen Steg (62, 62") miteinander verbunden sind.

2. Videoendoskop nach Anspruch 1, **dadurch gekennzeichnet, dass** die zumindest eine Leiterplatte (40; 40'; 40") eine Mehrzahl an Leiterbahnen (40a-d) aufweist, die nebeneinander und höchstens in zwei Ebenen der Leiterplatte (40; 40'; 40") angeordnet sind.

3. Videoendoskop nach Anspruch 1 oder 2, **dadurch gekennzeichnet, dass** die zweite Leiterplatte (41; 41") eine Mehrzahl an Leiterbahnen (40a-d) aufweist, die nebeneinander und in höchstens zwei Ebenen der zweiten Leiterplatte (41; 41") angeordnet sind.

4. Videoendoskop nach einem der Ansprüche 1 bis 3, **dadurch gekennzeichnet, dass** sich an die zumindest eine Leiterplatte (40; 40'; 40") proximalseitig zumindest ein proximales, vorzugsweise starres, Leiterplattenteil (48; 48'; 48") anschließt, das elektrische Bauteile (50; 50'; 50") trägt.

5. Videoendoskop nach Anspruch 4, **dadurch gekennzeichnet, dass** das proximale Leiterplattenteil (48a") mit einem weiteren proximalen, vorzugsweise starren, Leiterplattenteil (48b"), das elektrische Bauteile (50b") trägt, über einen flexiblen Steg (49) verbunden ist.

6. Videoendoskop nach Anspruch 5, **dadurch gekennzeichnet, dass** die proximalen Leiterplattenteile (48a", 48b") im Handstück (16') übereinanderliegend angeordnet sind.

7. Videoendoskop nach einem der Ansprüche 4 bis 6, **dadurch gekennzeichnet, dass** das zumindest eine proximale Leiterplattenteil (48; 48'; 48") den elektrischen Anschluss (38; 38'; 38") aufweist.

8. Videoendoskop nach einem der Ansprüche 1 bis 7, **dadurch gekennzeichnet, dass** die zumindest eine Leiterplatte (40; 40'; 40") an ihrem distalen Ende ein distales, vorzugsweise starres, Leiterplattenteil (44; 44'; 44") zur Kontaktierung mit dem Bildaufnehmer (34; 34') aufweist.

9. Videoendoskop nach Anspruch 8, **dadurch gekennzeichnet, dass** das distale Leiterplattenteil (44; 44'; 44") dicker und/oder breiter als die sich an das distale Leiterplattenteil (44; 44'; 44") anschließende Leiterplatte (40; 40'; 40") ist.

10. Videoendoskop nach Anspruch 8 oder 9, **dadurch gekennzeichnet, dass** die Breite des distalen Leiterplattenteils (44; 44'; 44") höchstens so groß ist wie die Breite des Bildaufnehmers (34; 34').

11. Videoendoskop nach einem der Ansprüche 1 bis 10, **dadurch gekennzeichnet, dass** die Flexibilität der zumindest einen Leiterplatte (40; 40'; 40") von proximal nach distal kontinuierlich oder in Stufen abnimmt.

12. Videoendoskop nach einem der Ansprüche 1 bis 11, **dadurch gekennzeichnet, dass** die Breite der zumindest einen Leiterplatte (40; 40'; 40") höchstens so groß ist wie die Breite des Bildaufnehmers (34; 34').

13. Videoendoskop nach einem der Ansprüche 1 bis 12, **dadurch gekennzeichnet, dass** die zumindest eine Leiterplatte (40; 40'; 40") einstückig aus einem planaren Trägermaterial gefertigt ist.

14. Videoendoskop nach einem der Ansprüche 1 bis 3, **dadurch gekennzeichnet, dass** die erste und zweite Leiterplatte (40', 41; 40", 41") und der sie verbindende Steg (62; 62") einstückig aus einem planaren Trägermaterial gefertigt sind.

15. Videoendoskop nach einem der Ansprüche 4 bis 7, **dadurch gekennzeichnet, dass** die zumindest eine Leiterplatte (40; 40'; 40") und das zumindest eine proximale Leiterplattenteil (48; 48'; 48") einstückig aus einem planaren Trägermaterial aufgebaut sind.

16. Videoendoskop nach einem der Ansprüche 4 bis 7, **dadurch gekennzeichnet, dass** die zumindest eine flexible Leiterplatte (40; 40'; 40") und das zumindest eine proximale Leiterplattenteil (48; 48'; 48") verschiedenen Trägermaterialien oder aus dem gleichen Trägermaterial mit unterschiedlichen Flexibilitäten gefertigt sind.

17. Videoendoskop nach einem der Ansprüche 8 bis 10, **dadurch gekennzeichnet, dass** die zumindest eine Leiterplatte (40; 40'; 40") und das zumindest eine distale Leiterplattenteil (44; 44'; 44") einstückig aus einem planaren Trägermaterial gefertigt sind.

18. Videoendoskop nach einem der Ansprüche 8 bis 10, **dadurch gekennzeichnet, dass** die zumindest eine Leiterplatte (40; 40'; 40") und das zumindest eine distale Leiterplattenteil (44; 44'; 44") aus verschiedenen Trägermaterialien oder aus dem gleichen Trägermaterial mit unterschiedlichen Flexibilitäten gefertigt sind.

19. Videoendoskop nach einem der Ansprüche 13 bis 18, **dadurch gekennzeichnet, dass** das Trägermaterial oder die Trägermaterialien ein polymerer Kunststoff, insbesondere Polyimid, ist.

20. Videoendoskop nach einem der Ansprüche 1 bis 19, **dadurch gekennzeichnet, dass** die zumindest eine Leiterplatte (40; 40'; 40") aus einer Mehrzahl von Leiterplattenabschnitten aufgebaut ist, die unterschiedliche Flexibilitäten aufweisen.

21. Videoendoskop nach einem der Ansprüche 1 bis 20, **dadurch gekennzeichnet, dass** die zumindest eine Leiterplatte (40; 40'; 40") in ihrem flexiblen Bereich aus mehreren, vorzugsweise zwei Leiterplattenlagen aufgebaut ist.

## Claims

1. A video endoscope, comprising an elongate shaft (12; 12') having a longitudinal axis (14; 14'), and a handpiece (16; 16') at the proximal end of the shaft (12; 12'), a distal end of the shaft (12; 12') being provided with a lens (24; 24') and, in the proximal direction from the lens (24; 24'), with an electronic image pickup (34; 34'), the image pickup (34; 34') being connected, via an electrical connection (36; 36'; 36") extending along the shaft (12; 12'), to an electrical connector piece (38; 38') arranged on the handpiece (16; 16'), and the image pickup (34; 34') and the connector piece (38; 38') being able to rotate relative to each other about the longitudinal axis (14; 14') of the shaft (12; 12'), **characterized in that** the electrical connection (36; 36'; 36") comprises at least one flexible, elongate and narrow circuit board (40; 40'; 40"), which is able to twist about its longitudinal direction and has at least one conductor track (40a-d), that the electrical connection (36'; 36") has a second flexible, elongate and narrow circuit board (41; 41") which is able to twist about its longitudinal direction and has at least one conductor track (40a-d), and that the first and second circuit boards (40', 41; 40", 41") are arranged lying one over the other in the shaft (12') and are movable relative to each other, wherein the first and second circuit boards (40', 41; 40", 41") are connected to each other in the area of their proximal end via a flexible bridge (62, 62").

2. The video endoscope of claim 1, **characterized in that** the at least one circuit board (40; 40'; 40") has a plurality of conductor tracks (40a-d) that are arranged next to one another and at most in two planes of the circuit board (40; 40'; 40").

3. The video endoscope of claim 1 or 2, **characterized in that** the second circuit board (41; 41") has a plurality of conductor tracks (40a-d) which are arranged next to one another and in at most two planes of the second circuit board (41; 41 ").

4. The video endoscope of any one of claims 1 through 3, **characterized in that** the at least one circuit board (40; 40'; 40") is adjoined proximally by at least one proximal and preferably rigid circuit board part (48; 48'; 48"), which carries electrical components (50; 50'; 50").

5. The video endoscope of claim 4, **characterized in that** the proximal circuit board part (48a") is connected by a flexible bridge (49) to another proximal and preferably rigid circuit board part (48b"), which carries electrical components (50b").

6. The video endoscope of claim 5, **characterized in that** the proximal circuit board parts (48a"; 48b") are arranged lying one over the other in the handpiece (16').

7. The video endoscope of any one of claims 4 through 6, **characterized in that** the at least one proximal circuit board part (48; 48'; 48") comprises the electrical connector piece (38; 38'; 38").

8. The video endoscope of any one of claims 1 through 7, **characterized in that**, at its distal end, the at least one circuit board (40; 40'; 40") has a distal and preferably rigid circuit board part (44; 44', 44") for contact with the image pickup (34; 34').

9. The video endoscope of claim 8, **characterized in that** the distal circuit board part (44; 44'; 44") is thicker and/or wider than the circuit board (40; 40'; 40") adjoining the distal circuit board part (44; 44'; 44").

10. The video endoscope of claim 8 or 9, **characterized in that** the width of the distal circuit board part (44; 44'; 44") is at most as great as the width of the image pickup (34; 34').

11. The video endoscope of any one of claims 1 through 10, **characterized in that** the flexibility of the at least one circuit board (40; 40'; 40") decreases continuously or in steps from the proximal end to the distal end.

12. The video endoscope of any one of claims 1 through 11, **characterized in that** the width of the at least one circuit board (40; 40'; 40") is at most as great as the width of the image pickup (34; 34').

13. The video endoscope of any one of claims 1 through 12, **characterized in that** the at least one circuit board (40; 40'; 40") is produced in one piece from a planar base material.

14. The video endoscope of any one of claims 1 through 3, **characterized in that** the first and second circuit boards (40', 41; 40", 41") and the bridge (62; 62") connecting them are produced in one piece from a planar base material.

15. The video endoscope of any one of claims 4 through 7, **characterized in that** the at least one circuit board (40; 40'; 40") and the at least one proximal circuit board part (48; 48'; 48") are constructed in one piece from a planar base material.

16. The video endoscope of any one of claims 4 through 7, **characterized in that** the at least one flexible circuit board (40; 40'; 40") and the at least one proximal circuit board part (48; 48'; 48") are produced from different base materials or from the same base material with different degrees of flexibility.

17. The video endoscope of any one of claims 8 through 10, **characterized in that** the at least one circuit board (40; 40'; 40") and the at least one distal circuit board part (44; 44'; 44") are produced in one piece from a planar base material.

18. The video endoscope of any one of claims 8 through 10, **characterized in that** the at least one circuit board (40; 40'; 40") and the at least one distal circuit board part (44; 44'; 44") are produced from different base materials or from the same base material with different degrees of flexibility.

19. The video endoscope of any one of claims 13 through 18, **characterized in that** the base material or base materials is/are a polymer plastic, in particular polyimide.

20. The video endoscope of any one of claims 1 through 19, **characterized in that** the at least one circuit board (40; 40'; 40") is constructed from a plurality of circuit board sections that have different degrees of flexibility.

21. The video endoscope of any one of claims 1 through 20, **characterized in that** the at least one circuit board (40; 40'; 40") is constructed, in its flexible area, from several circuit board layers, preferably from two circuit board layers.

## Revendications

1. Vidéo-endoscope, comportant une tige (12 ; 12') allongée, munie d'un axe longitudinal (14 ; 14'), et une poignée (16 ; 16') à l'extrémité proximale de la tige (12 ; 12'), un objectif (24 ; 24') étant disposé à une extrémité distale de la tige (12 ; 12'), et une caméra (34 ; 34') électronique étant disposée sur le côté proximal de l'objectif (24 ; 24'), la caméra (34 ; 34') étant reliée, via une liaison électrique (36 ; 36' ; 36") s'étendant le long de la tige (12 ; 12'), à un raccord (38 ; 38') électrique, agencé sur la poignée (16 ; 16'), et la caméra (34 ; 34') et le raccord (38 ; 38') étant aptes à tourner l'un par rapport à l'autre autour de l'axe longitudinal (14 ; 14') de la tige (12 ; 12'), **caractérisé en ce que** la liaison électrique (36 ; 36' ; 36") comporte une plaquette de circuits imprimés (40, 40' ; 40") allongée, étroite, flexible, apte à tourner autour de sa direction longitudinale, laquelle comporte au moins une piste conductrice (40a-d), **en ce que** la liaison électrique (36' ; 36") comporte une deuxième plaquette de circuits imprimés (41 ; 41") allongée, étroite, flexible, apte à tourner autour de sa direction longitudinale, laquelle comporte au moins une piste conductrice (40a-d), et **en ce que** la première et la deuxième plaquette de circuits imprimés (40', 41 ; 40", 41") sont disposées dans la tige (12 ; 12') l'une au-dessus de l'autre et de manière mobile l'une par rapport à l'autre, la première et la deuxième plaquette de circuits imprimés (40', 41 ; 40", 41") étant reliées l'une à l'autre par l'intermédiaire d'une barrette (62, 62") flexible dans la zone de leur extrémité proximale.

2. Vidéo-endoscope selon la revendication 1, **caractérisé en ce que** ladite au moins une plaquette de circuits imprimés (40 ; 40' ; 40") comporte une pluralité de pistes conductrices (40a-d), qui sont disposées les unes à côté des autres et au maximum dans deux plans de la plaquette de circuits imprimés (40 ; 40' ; 40").

3. Vidéo-endoscope selon la revendication 1 ou 2, **caractérisé en ce que** la deuxième plaquette de circuits imprimés (41 ; 41 ") comporte une pluralité de pistes conductrices (40a-d), qui sont disposées les unes à côté des autres et au maximum dans deux plans de la deuxième plaquette de circuits imprimés (41 ; 41").

4. Vidéo-endoscope selon l'une quelconque des revendications 1 à 3, **caractérisé en ce qu'**au moins une partie proximale (48 ; 48' ; 48"), de préférence rigide, qui porte des composants électriques (50 ; 50' ; 50"), est adjacente du côté proximal à ladite au moins une plaquette de circuits imprimés (40 ; 40' ; 40").

5. Vidéo-endoscope selon la revendication 4, **caractérisé en ce que** la partie proximale (48a") est reliée, par l'intermédiaire d'une barrette (49) flexible, à une autre partie proximale (48b"), de préférence rigide, laquelle porte des composants électriques (50b").

6. Vidéo-endoscope selon la revendication 5, **caractérisé en ce que** les parties proximales (48a", 48b") sont disposées l'une au-dessus de l'autre dans la poignée (16').

7. Vidéo-endoscope selon l'une quelconque des revendications 4 à 6, **caractérisé en ce que** ladite au moins une partie proximale (48 ; 48' ; 48") comporte le raccord (38 ; 38' ; 38") électrique.

8. Vidéo-endoscope selon l'une quelconque des revendications 1 à 7, **caractérisé en ce que** ladite au moins une plaquette de circuits imprimés, (40 ; 40' ; 40") comporte, au niveau de son extrémité distale, une partie distale (44 ; 44' ; 44"), de préférence rigide, pour la mise en contact avec la caméra (34 ; 34').

9. Vidéo-endoscope selon la revendication 8, **caractérisé en ce que** la partie distale (44 ; 44' ; 44") est plus épaisse et/ou plus large que la plaquette de circuits imprimés (40 ; 40' ; 40") adjacente à la partie distale (44 ; 44' ; 44").

10. Vidéo-endoscope selon la revendication 8 ou 9, **caractérisé en ce que** la largeur de la partie distale (44 ; 44' ; 44") est au maximum aussi grande que la largeur de la caméra (34 ; 34').

11. Vidéo-endoscope selon l'une quelconque des revendications 1 à 10, **caractérisé en ce que** la flexibilité de la plaquette de circuits imprimés (40 ; 40' ; 40") diminue de proximal en distal en continu ou par paliers.

12. Vidéo-endoscope selon l'une quelconque des revendications 1 à 11, **caractérisé en ce que** la largeur de ladite au moins une plaquette de circuits imprimés (40 ; 40' ; 40") est au maximum aussi grande que la largeur de la caméra (34 ; 34').

13. Vidéo-endoscope selon l'une quelconque des revendications 1 à 12, **caractérisé en ce que** ladite au moins une plaquette de circuits imprimés (40 ; 40' ; 40") est réalisée d'un seul tenant dans un matériau support planaire.

14. Vidéo-endoscope selon l'une quelconque des revendications 1 à 3, **caractérisé en ce que** la première et la deuxième plaquette de circuits imprimés (40', 41' ; 40", 41") et la barrette (62, 62") qui les relie sont réalisées d'un seul tenant dans un matériau support planaire.

15. Vidéo-endoscope selon l'une quelconque des revendications 4 à 7, **caractérisé en ce que** ladite au moins une plaquette de circuits imprimés (40 ; 40' ; 40") et ladite au moins une partie proximale (48 ; 48' ; 48") sont réalisées d'un seul tenant dans un matériau support planaire.

16. Vidéo-endoscope selon l'une quelconque des revendications 4 à 7, **caractérisé en ce que** ladite au moins une plaquette de circuits imprimés (40 ; 40' ; 40") flexible et ladite au moins une partie proximale (48 ; 48' ; 48") sont réalisées dans des matériaux support différents ou dans le même matériau support avec des flexibilités différentes.

17. Vidéo-endoscope selon l'une quelconque des revendications 8 à 10, **caractérisé en ce que** ladite au moins une plaquette de circuits imprimés (40 ; 40' ; 40") et ladite au moins une partie distale (44 ; 44' ; 44") sont réalisées d'un seul tenant dans un matériau support planaire.

18. Vidéo-endoscope selon l'une quelconque des revendications 8 à 10, **caractérisé en ce que** ladite au moins une plaquette de circuits imprimés (40 ; 40' ; 40") et ladite au moins une partie distale (44 ; 44' ; 44") sont réalisées dans des matériaux support différents ou dans le même matériau support avec des flexibilités différentes.

19. Vidéo-endoscope selon l'une quelconque des revendications 13 à 18, **caractérisé en ce que** le matériau support ou les matériaux support sont une matière plastique polymère, en particulier un polyimide.

20. Vidéo-endoscope selon l'une quelconque des revendications 1 à 19, **caractérisé en ce que** ladite au moins une plaquette de circuits imprimés (40 ; 40' ; 40") est constituée d'une pluralité de portions qui présentent des flexibilités différentes.

21. Vidéo-endoscope selon l'une quelconque des revendications 1 à 20, **caractérisé en ce que** ladite au moins une plaquette de circuits imprimés (40 ; 40' ; 40"), dans sa zone flexible, est constituée de plusieurs couches, de préférence deux couches.
